# EUROPÄISCHE PATENTSCHRIFT

(11) **EP 3 643 399 B1**
(45) Veröffentlichungstag und Bekanntmachung des Hinweises auf die Patenterteilung: **27.04.2022**
(21) Anmeldenummer: 19190458.0
(22) Anmeldetag: 07.08.2019
(51) Int. Cl.: B01F 33/501, B01F 35/71, B01F 35/75

(54) **VORRICHTUNG UND VERFAHREN ZUM BEREITSTELLEN VON KNOCHENZEMENT**
DEVICE AND METHOD FOR PRODUCING BONE CEMENT
DISPOSITIF ET PROCÉDÉ DE FOURNITURE DE CIMENT OSSEUX

(30) Priorität: 25.10.2018 DE 102018218303; 25.10.2018 DE 102018218302
(43) Veröffentlichungstag der Anmeldung: 29.04.2020
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(56) Entgegenhaltungen:
- WO-A1-2006/123205
- WO-A1-2011/089480
- US-A1- 2014 269 147

## Beschreibung

### Beschreibung

Die Erfindung betrifft ein Verfahren zur Herstellung eines Knochenzements aus wenigstens zwei Ausgangskomponenten in einer Vorrichtung aufweisend ein erstes hohlzylinderförmiges Behältnis und ein zweites hohlzylinderförmiges Behältnis, ein in dem ersten Behältnis angeordnetes Gefäß, wobei das Gefäß eine Monomerflüssigkeit lagert. Die Erfindung betrifft weiterhin eine Vorrichtung zum Bereitstellen eines Knochenzements aus zwei Ausgangskomponenten, aufweisend ein hohlzylinderförmiges erstes Behältnis, in dem ein Gefäß für eine Monomerflüssigkeit als erste Ausgangskomponente lagerbar ist, ein hohlzylinderförmiges zweites Behältnis umfassend einen ersten Innenraum, in dem ein Knochenzementpulver als zweite Ausgangskomponente lagerbar ist, und einen zweiten Innenraum, in den die Monomerflüssigkeit förderbar ist, ein fluidleitendes Fördermittel, angeordnet zwischen dem ersten Innenraum und dem zweiten Innenraum, wobei das erste Behältnis und das zweite Behältnis axial miteinander verbunden sind.

### Hintergrund der Erfindung

Es werden erhebliche Bemühungen unternommen, Vorrichtungen und Verfahren zum Bereitstellen von Knochenzement aufzuzeigen, mittels derer Knochenzement einfach, sicher und schnell bereitgestellt werden kann. Ein wichtiger Aspekt bei der Bereitstellung von Knochenzement ist die Vermeidung von Lufteinschlüssen im Knochenzement. Zu deren Vermeidung wurden eine Vielzahl von Vakuum-Zementiersystemen beschreiben, von denen exemplarisch folgende genannt sind: US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzement. Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden. Solche geschlossenen, sogenannten Full-Prepacked-Systeme, sind in folgenden Schriften genannt: EP 0692229 A1, DE 10 2009 031 178 B3, US 5,997,544 A, US 6,709,149 B1, DE 698 12 726 T2, EP 0796653 A2, US 5,588,745 A.

In der DE 10 2016 121 607 A1 wird ein Full-Prepack-System beschrieben, wobei der mit Monomerflüssigkeit gefüllte Behälter axial hinter dem Knochenzement gelagert. Nachteilig ist, dass der mit Monomerflüssigkeit gefüllte Behälter im Zuge des Mischens der Ausgangkomponenten vollständig zerstört werden muss. Als ebenso nachteilig hat sich herausgestellt, dass der Krafteintrag zum Mischen der Ausgangskomponenten als auch später beim Austragen des gemischten Knochenzements immer auch auf die Überreste des zerstörten Behälters einwirkt. Dies führt zu einem erhöhten Kraftaufwand beim Anwender als auch zu ruckartigen und unkontrollierbaren Bewegungen sowohl im Zuge der Zerstörung der Behälter, als auch im Zuge des Mischens und späteren Austragens des gemischten Zements. Beides erschwert in erheblichem Maße die Anwendbarkeit des Full-Prepack-Systems, insbesondere im Zuge von zeitkritischen Operationsbedingungen. Als ebenso nachteilig hat sich herausgestellt, dass der zerstörte Behälter die Verwendung des Full-Prepack-Systems erschwert. Dies geschieht beispielsweise durch Bruchstücke des Behälters, die Monomerflüssigkeit zurückhalten, welche dann nicht zur Herstellung des Knochenzements zur Verfügung steht. Ein weiterer Nachteil entsteht durch Verkeilungen der Überreste des Behälters innerhalb der Misch - und Austragsvorrichtung. Ein weiterer Nachteil ist das Gefährdungspotential der Bruchstücke sowohl für den Anwender, als auch für den Patienten.

Aus der WO-A1-2006/123205 wird ein Verfahren und eine Vorrichtung bekannt, wobei in einer Vorrichtung aufweisend ein erstes hohlzylinderförmiges Behältnis und ein zweites hohlzylinderförmiges Behältnis, ein in dem ersten Behältnis angeordnetes Gefäß, wobei das Gefäß eine Monomerflüssigkeit lagert, das Öffnen des Gefäßes mittels einer ersten Bewegung des Gefäßes relativ zum zweiten Behältnis erzeugt wird.

### Aufgaben

Eine Aufgabe der vorliegenden Erfindung ist es, einen oder mehrere der sich aus dem Stand der Technik ergebenden Nachteile zumindest teilweise zu überwinden.

Im Speziellen basiert die Erfindung auf dem Ziel, Vorrichtungen zum Bereitstellen eines Knochenzements aus zwei Ausgangskomponenten mit einem durch den Anwender geringeren benötigten Krafteinwirkung als bisherige Zementiersysteme bereitzustellen, welche zudem einfach handhabbar sowie sicher in ihrer Anwendung sind. Das Ziel ist zudem, die Infektionsgefahr für den Patienten zu minimieren. Die Vorrichtungen sollen geeignet sein, die zwei Ausgangskomponenten separat voneinander lagern zu können. Die zwei Ausgangskomponenten sollen in der Vorrichtung innerhalb weniger Sekunden in der geschlossenen Vorrichtung zusammengeführt werden können. Die Vorrichtung soll den Knochenzement ohne eine mechanische Durchmischung der Ausgangskomponenten bereitstellen. Die Vorrichtung soll weiterhin so ausgestaltet sein, dass der Anwender keine Montageschritte durchführen muss. Die Vorrichtung soll ohne extern angelegtes Vakuum in der Lage sein den Knochenzement bereitzustellen. Die Vorrichtung soll den bereitgestellten Knochenzement austragen können. Die Vorrichtung soll den bereitgestellten Knochenzement ohne Umbaumaßnahmen austragen können. Die Vorrichtung soll ohne Umbaumaßnahmen und ohne externe Gerätschaften, wie beispielsweise Schläuche, Vakuumquellen oder Auspressvorrichtungen, den Knochenzement bereitstellen und austragen können. Die Vorrichtung soll mit möglichst wenigen Arbeitsschritte bedient werden können, um Fehlerquellen durch den Anwender zu minimieren.

Es ist eine weitere Aufgabe der Erfindung, ein Verfahren bereitzustellen, mit dem Knochenzement aus zwei Ausgangskomponenten bereitgestellt werden kann, mittels derer mindestens ein Teil der bereits beschriebenen Aufgaben zumindest zum Teil gelöst ist.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Werte Y und kleiner als Y.

### Ausführliche Beschreibung der Erfindung

Ein erster Gegenstand der Erfindung betrifft ein Verfahren zur Herstellung eines Knochenzements mit den Merkmalen des Anspruchs 1.

Da das mit der Monomerflüssigkeit gefüllte Gefäß innerhalb des ersten Behältnisses gelagert ist, und das erste Behältnis im Zuge des Verfahrens mittels Einschieben in das zweite Behältnis sowohl die Monomerflüssigkeit, als auch später den Knochenzement fördert, ist ein vollständiges Zerstören des Gefäßes nicht notwendig. Das Verfahren ermöglicht daher, dass das Gefäß nur punktuell geöffnet werden muss. Die Folge ist ein geringerer Kraftaufwand für den Anwender und zugleich ein kontrolliertes Fördern der Monomerflüssigkeit in den ersten Innenraum, da keine Bruchstücke des Gefäßes im Verschiebeweg des Austragskolbens liegen. Zudem besteht ein geringeres Risiko einer Verkeilung durch Bruchstücke des Gefäßes während der Verwendung der Vorrichtung. Ebenso ist das Verletzungsrisiko durch Bruchstücke sowohl für den Patienten, als auch für den Anwender, gesenkt. Des Weiteren werden im Zuge des Verfahrens keine Umbaumaßnahmen an der Vorrichtung, sowie keine externen Gerätschaften wie beispielsweise Pumpen oder Schläuche, benötigt. Dies sorgt zusätzlich zu den bereits oben beschriebenen Vorteilen des punktuellen Öffnens des Gefäßes für einen einfachen, sicheren und schnellen Ablauf des Verfahrens. Sowohl das Fördern der Monomerflüssigkeit, als auch das Austragen des Knochenzements, werden sequentiell mit einer gleichartigen Verschiebung bewerkstelligt. Somit ist sichergestellt, dass einem Anwender keine Verwechslung von Verfahrensschritten unterlaufen kann. Die Reihenfolge der einzelnen Schritte ist vielmehr von der Vorrichtung vorgegeben.

Eine Ausgestaltungsform des erfindungsgemäßen Verfahrens ist dadurch gekennzeichnet, dass zum Öffnen gemäß Schritt a) das Gefäß auf eine im ersten Behältnis angeordnete Öffnungsvorrichtung gepresst wird. Erfindungsgemäß ist unter einer Öffnungsvorrichtung eine Vorrichtung zu verstehen, welche dazu geeignet ist, die strukturelle Integrität des Gefäßes zu zerstören und damit zu öffnen. Erfindungsgemäß ist die Ausgestaltungsform der Öffnungsvorrichtung in Abhängigkeit der strukturellen Stabilität des Gefäßes zu wählen. Das Material der Öffnungsvorrichtung ist erfindungsgemäß dazu in der Lage, die strukturelle Integrität des Materials des Gefäßes zu zerstören. Es ist vorteilhaft, falls die Öffnungsvorrichtung im Vergleich zur zu öffnenden Fläche des Gefäßes einen kleinen Durchmesser aufweist. Dadurch wird der benötigte Kraftaufwand zum Öffnen des Gefäßes reduziert. Beispielsweise kann das Öffnen des Gefäßes durch Einstechen oder Einschneiden erfolgen. Beispiele für Öffnungsvorrichtungen umfassen Anstechdorne, Nadeln, Kanülen und Schneidkanten.

In einer bevorzugten Ausgestaltungsform ist das Gefäß eine Glasampulle und die Öffnungsvorrichtung ein Anstechdorn. Der Antechdorn hat den Vorteil, dass er die Glasampulle punktuell öffnen kann, den Rest der Ampulle jedoch intakt lässt. In einer weiter bevorzugten Ausgestaltungsform ist das Gefäß eine Glasampulle und die Öffnungsvorrichtung ein Anstechdorn, angeordnet derart, dass der Anstechdorn einen Ampullenboden der Glasampulle öffnet. Eine typische Glasampulle weist einen Ampullenkörper, einen Ampullenkopf und einen Ampullenboden auf. Der Ampullenkopf zeichnet sich durch einen, im Vergleich zum Ampullenkörper, kleinen Durchmesser aus, welcher als eine Sollbruchstelle fungiert. Typischerweise wird die Glasampulle an der Sollbruchstelle geöffnet. Der Ampullenboden hat typischerweise den gleichen Durchmesser wie der Ampullenkörper. Erfindungsgemäß ist es von Vorteil, wenn die Glasampulle am Ampullenboden aufgebrochen wird. Durch das Anstechen am Ampullenboden mittels Anstechdorn verläuft zum einen die Öffnung der Glasampulle durch einen großen Flächenunterschied von Öffnungsvorrichtung zu Ampullenboden kontrollierter. Zum anderen bricht der Ampullenkopf durch das Öffnen nicht vom restlichen Ampullenkörper ab, wodurch sowohl der Kraftaufwand des Öffnens als auch die Anzahl der Bruchstücke reduziert wird.

Eine weitere Ausgestaltungsform des erfindungsgemäßen Verfahrens ist dadurch gekennzeichnet, dass zum Öffnen gemäß Schritt a) das Gefäß mittels eines im ersten Behältnis angeordneten Förderkolbens auf die Öffnungsvorrichtung gepresst wird. Erfindungsgemäß ist unter einem Förderkolben ein axial bewegbares Bauteil zu verstehen, welches durch eine gezielte axiale Verschiebung innerhalb des ersten Behältnisses das Gefäß auf die Öffnungsvorrichtung pressen und dadurch öffnen kann. Erfindungsgemäß ist das Gefäß zwischen Öffnungsvorrichtung und Förderkolben gelagert. Der Förderkolben kann auf der dem Gefäß zugewandten Seite unterschiedliche Formen annehmen. Beispielsweise kann der Förderkolben flach sein oder als ein Schulterelement ausgeformt sein. Ist das Gefäß eine Ampulle, bevorzugt eine Glasampulle, ist der Förderkolben bevorzugt als Schulterelement ausgeformt. Wie bereits oben beschrieben, ist es von Vorteil, falls die Glasampulle am Ampullenboden geöffnet wird. Entsprechend weist der Ampullenkopf in Richtung des Förderkolbens. Ein Schulterelement ist bevorzugt, da das Schulterelement nicht am Ampullenkopf, sondern am Ampullenkörper, ansetzt. Das Schulterelement ist dabei so ausgestaltet, ein Kontakt nur mit einer Schulter, nicht aber mit dem Ampullenkopf des Gefäßes zustande kommt. Das Schulterelement ist dabei rohrartig ausgeformt, wobei der Hohlraum einen derart großen Durchmesser aufweist, dass der Ampullenkopf, nicht aber die Schulter des Gefäßes in das Schulterelement aufgenommen werden kann. Somit schützt das Schulterelement den Ampullenkopf vor einem unkontrollierten Abbrechen. Dadurch wirkt die eingebrachte Kraft des Förderkolbens nicht auf die Sollbruchstelle und der Ampullenkopf verbleibt am Ampullenkörper mit den bereits oben beschriebenen Vorteilen.

Eine weitere Ausgestaltungsform des erfindungsgemäßen Verfahrens ist dadurch gekennzeichnet, dass an dem ersten Behältnis ein Schraubmittel angeordnet ist, welches kraft- und/oder formschlüssig auf den Förderkolben wirkt, um das Gefäß in der ersten Bewegung auf die Öffnungsvorrichtung zu pressen. Vorzugsweise kann das Schraubmittel als Griff ausgestaltet sein, so dass der Krafteintrag für den Anwender leichter aufzubringen ist. In einer Ausgestaltungsform kann das Schraubmittel ein Innengewinde und das erste Behältnis ein Außengewinde aufweisen, wobei die Gewinde form- und/oder kraftschlüssig zusammenwirken, um das Gefäß mittels des Förderkolbens auf die Öffnungsvorrichtung zu schieben.

Erfindungsgemäß fließt nach dem Öffnen des Gefäßes die Monomerflüssigkeit in einen zweiten Innenraum in dem zweiten Behältnis. Dafür wird die Vorrichtung nach erfolgtem Öffnen des Gefäßes so gehalten, dass das erste Behältnis räumlich höher als das zweite Behältnis angeordnet ist. Dadurch minimiert das erfindungsgemäße Verfahren mögliche Fehlerquellen und Anwendungsfehler, da zum Fördern der Monomerflüssigkeit in den zweiten Innenraum nur das Gefäß geöffnet und die Vorrichtung gemäß oben beschriebener Ausrichtung gehalten werden muss. Eine zusätzliche Umbaumaßnahme oder zusätzlicher Arbeitsschritt wie beispielsweise das Umlegen eines Hebels ist nicht notwendig.

Erfindungsgemäß weist das erste Behältnis einen Austragskolben auf, welcher bei der ersten Verschiebung in den zweiten Innenraum bewegt wird. Der Austragskolben ist zwischen erstem Behältnis und zweitem Innenraum angeordnet. Dies erlaubt eine direkte, nicht auf das Gefäß einwirkende, Krafteinwirkung auf die in den zweiten Innenraum geförderte Monomerflüssigkeit durch eine axiale Verschiebung des Austragskolbens. Dadurch muss, wie bereits oben beschrieben, nach einem Öffnen des Gefäßes und Ausfließen der Monomerflüssigkeit, das Gefäß nicht vollständig zerstört werden.

Erfindungsgemäß fördert der Austragskolben bei der ersten Verschiebung die Monomerflüssigkeit in das Knochenzementpulver und auspresst bei der zweiten Verschiebung den Knochenzement aus der Vorrichtung. Die räumliche Lage des Austragskolbens zwischen erstem Behältnis und zweitem Innenraum hat zur Folge, dass der Austragskolben, im Zusammenspiel mit der Behältniswand, zunächst für Gase und Flüssigkeiten durchlässig ausgestaltet sein muss, damit die Monomerflüssigkeit in den zweiten Innenraum gelangen kann. In der Folge muss der Austragskolben für Flüssigkeiten und Feststoffe undurchlässig ausgestaltet sein, da sonst ein Fördern der Monomerflüssigkeit aus dem zweiten Innenraum in den ersten Innenraum nicht möglich ist. Erfindungsgemäß durchläuft der Austragskolben im Zuge der Verwendung der Vorrichtung eine Transformation von für Gase und Flüssigkeiten durchlässig zu für Flüssigkeiten und Feststoffe undurchlässig. Unter einer Position des Austragskolbens ist erfindungsgemäß ein Zustand des Austragskolbens oder eine räumliche Anordnung des Austragskolbens in dem zweiten Behältnis zu verstehen.

In einer Ausgestaltungsform weist der Austragskolben in der ersten Position mindestens eine Öffnung auf, welche den Austausch von Gasen und Flüssigkeiten zwischen dem ersten Behältnis und dem zweiten Innenraum erlaubt. Entsprechend ist die mindestens eine Öffnung in der zweiten Position des Austragskolbens für flüssige und feste Materie verschlossen. Beispielsweise kann der Austragskolben in der ersten Position mindestens eine Öffnung aufweisen, welche durch eine Drehung des Austragskolbens um die Längsachse des Austragskolbens in die zweite Position verschlossen werden, so dass kein Austausch mehr stattfindet.

Ein zweiter Gegenstand der vorliegenden Erfindung betrifft eine Vorrichtung mit den Merkmalen des Anspruchs 7.

Eine weitere Ausgestaltungsform der erfindungsgemäßen Vorrichtung ist dadurch gekennzeichnet, dass das erste Behältnis eine Öffnungsvorrichtung aufweist, um das Gefäß zu öffnen und ein Ausfließen der Monomerflüssigkeit zu ermöglichen.

Eine weitere Ausgestaltungsform der erfindungsgemäßen Vorrichtung ist dadurch gekennzeichnet, dass das erste Behältnis einen axial verschiebbaren Förderkolben aufweist, welcher kraft- und/oder formschlüssig auf das Gefäß wirkt, um das Gefäß mittels einer ersten Bewegung des Gefäßes auf die Öffnungsvorrichtung zu pressen.

Eine weitere Ausgestaltungsform der erfindungsgemäßen Vorrichtung ist dadurch gekennzeichnet, dass an dem ersten Behältnis ein Schraubmittel angeordnet ist, um den Förderkolben axial zu verschieben, insbesondere, dass das Schraubmittel an einem ersten Ende des ersten Behältnisses angeordnet ist.

Eine weitere Ausgestaltungsform der erfindungsgemäßen Vorrichtung ist dadurch gekennzeichnet, dass das Schraubmittel als ein Griff ausgestaltet ist, der zumindest teilweise das erste Behältnis haubenartig umfasst, insbesondere dass in der haubenartigen Umfassung ein Innengewinde angeordnet ist. Die Ausgestaltung als Griff ist für den Anwender von Vorteil, da so der vom Anwender benötigte Krafteintrag zum Öffnen des Gefäßes leichter aufzubringen ist.

Eine weitere Ausgestaltungsform der erfindungsgemäßen Vorrichtung ist dadurch gekennzeichnet, dass zumindest Teile einer Außenfläche des ersten Behältnisses ein Außengewinde aufweisen, welches mit dem Innengewinde der haubenartigen Umfassung kraft- und/oder formschlüssig zusammenwirkt. Das Zusammenwirken der Gewinde reduziert für den Anwender den aufzubringenden Krafteintrag und erleichtert somit die Verwendung der Vorrichtung.

Eine weitere Ausgestaltungsform der erfindungsgemäßen Vorrichtung ist dadurch gekennzeichnet, dass ein zweites Ende des ersten Behältnisses kolbenartig in das zweite Behältnis hineinragt. Das erste Behältnis besitzt dabei einen Außendurchmesser und das zweite Behältnisses einen Innendurchmesser, welche derart aufeinander abgestimmt sind, dass ein, zumindest teilweises Ineinanderschieben der Behältnisse gewährleistet ist. Durch das Hineinragen sind die Behältnisse miteinander verbunden oder verbindbar. In einer Ausgestaltungsform entspricht zumindest ein Teil des Außendurchmessers des ersten Behältnisses im Wesentlichen einem Innendurchmesser des zweiten Behältnisses, so dass das Ineinanderschieben der Behältnisse sowohl die Monomerflüssigkeit aus dem zweiten Innenraum in den ersten Innenraum, als auch, in einem späteren Verfahrensschritt, den Knochenzement aus der Vorrichtung fördern kann. In einer weiteren Ausgestaltungsform ist der Außendurchmesser zumindest zum Teil kleiner als der Innendurchmesser, so dass das Risiko eines Verkeilens der Behältnisse im Zuge des Ineinanderschiebens reduziert ist.

Eine weitere Ausgestaltungsform der erfindungsgemäßen Vorrichtung ist dadurch gekennzeichnet, dass das zweite Ende des ersten Behältnisses kraft- und/oder form- und/oder stoffschlüssig mit einem Austragskolben verbunden ist. Erfindungsgemäß wird darunter verstanden, dass das erste Behältnis und der Austragskolben fest miteinander verbunden sind oder das erste Behältnis und der Austragskolben aus nur einem Teil bestehen. In einer Ausgestaltungsform ist der Austragskolben beispielsweise mit einer Schraube, einem Nagel, einer Klemme, einem Keil oder einer Niete fest mit dem ersten Gehäuse verbunden. In einer weiteren Ausgestaltungsform ist das erste Behältnis so ausgeformt, dass eine Grundfläche des ersten Behältnisses den Austragskolben bildet. Sind das erste Behältnis und der Austragskolben kraft- und/oder form- und/oder stoffschlüssig miteinander verbunden, kann ein Krafteintrag auf das erste Behältnis direkt zum Fördern der Monomerflüssigkeit aus dem zweiten Innenraum in den ersten Innenraum genutzt werden. Ein Vorteil ist dabei, dass keine zusätzlichen Bauteile notwendig sind, um eine axiale Bewegung des Austragskolbens zu bewerkstelligen, was die Vorrichtung einfacherer und anwendungssicherer macht. Ein weiterer Vorteil ist, dass die Krafteinwirkung zum Verschieben des Austragskolbens axial auf die Vorrichtung eingebracht wird, was den Kraftaufwand beim Anwender reduziert.

Eine weitere Ausgestaltungsform der erfindungsgemäßen Vorrichtung ist dadurch gekennzeichnet, dass ein erstes Ende des zweiten Behältnisses ein Innengewinde aufweist. Durch das Innengewinde kann die axiale Verbindung zwischen erstem Behältnis und zweitem Behältnis hergestellt werden.

Eine weitere Ausgestaltungsform der erfindungsgemäßen Vorrichtung ist dadurch gekennzeichnet, dass zu einer zweiten Verschiebung das Innengewinde des zweiten Behältnisses mit dem Außengewinde des ersten Behältnisses kraft- und/oder formschlüssig zusammenwirkt, um den Knochenzement zu fördern. In einer Ausgestaltungsform wirken Innengewinde und Außengewinde derart zusammen, dass die zweite Verschiebung nur in die gewünschte Richtung ablaufen kann. Dies hat einerseits den Vorteil, dass ein Anwender keine zusätzliche Kraft aufwenden muss, um ein Zurückdrehen der Vorrichtung zu verhindern. Zum anderen wird ein Risiko der Bildung von Luftblasen reduziert.

Eine weitere Ausgestaltungsform der erfindungsgemäßen Vorrichtung ist dadurch gekennzeichnet, dass eine Gewindehülse zwischen dem Innengewinde und dem Außengewinde angeordnet ist, um eine Co-axiale doppelte Gewindepaarung zu bilden. Durch eine Verwendung der Gewindehülse kann das erste Behältnis mit einem geringeren Durchmesser als ohne Verwendung der Gewindehülse ausgebildet sein. Dies reduziert das Risiko einer Verkeilung zwischen erstem Behältnis und zweitem Behältnis bei der Verwendung der Vorrichtung.

Das erfindungsgemäße Verfahren ist dadurch gekennzeichnet, dass dieses Knochenzemente aus zwei Ausgangskomponenten herstellt. Erfindungsgemäß wird unter einem Knochenzement eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Bevorzugt handelt es sich bei Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzement, der auch als Knochenzementteig bezeichnet wird. Dabei wird eine Polymerisation des Monomers durch Radikale eingeleitet. Mit fortschreitender Polymerisation des Monomers erhöht sich die Viskosität des Knochenzements, bis dieser vollständig aushärtet.

Erfindungsgemäß wird unter einem Knochenzementpulver ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulvers zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen.

Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin.

Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

### Beispiele

Die Erfindung wird im Folgenden durch Beispiele weiter beispielhaft illustriert. Die Erfindung ist nicht auf die Beispiele beschränkt.

### Figuren

- Fig. 1: schematische Zeichnung eines Querschnitts einer Vorrichtung zum Bereitstellen eines Knochenzements aus zwei Ausgangskomponenten
- Fig. 2: die Vorrichtung aus Figur 1 um 90° nach rechts gedreht
- Fig. 3: die Vorrichtung aus Figur 2 beim Öffnen eines Gefäßes mit Monomerflüssigkeit
- Fig. 4: die Vorrichtung aus Figur 3 beim Fördern der Monomerflüssigkeit in einen ersten Innenraum
- Fig. 5: Vorrichtung aus Figur 4 bei der Ausbildung des Knochenzements
- Fig. 6: Vorrichtung aus Figur 5 beim Austragen des Knochenzements
- Fig. 7: Vorrichtung aus Figur 6 nach Beendigung des Austragevorgangs
- Fig. 8: Flussdiagramm (Verfahren zur Bereitstellung eines Knochenzements aus zwei Ausgangskomponenten)

### Beschreibung der Figuren

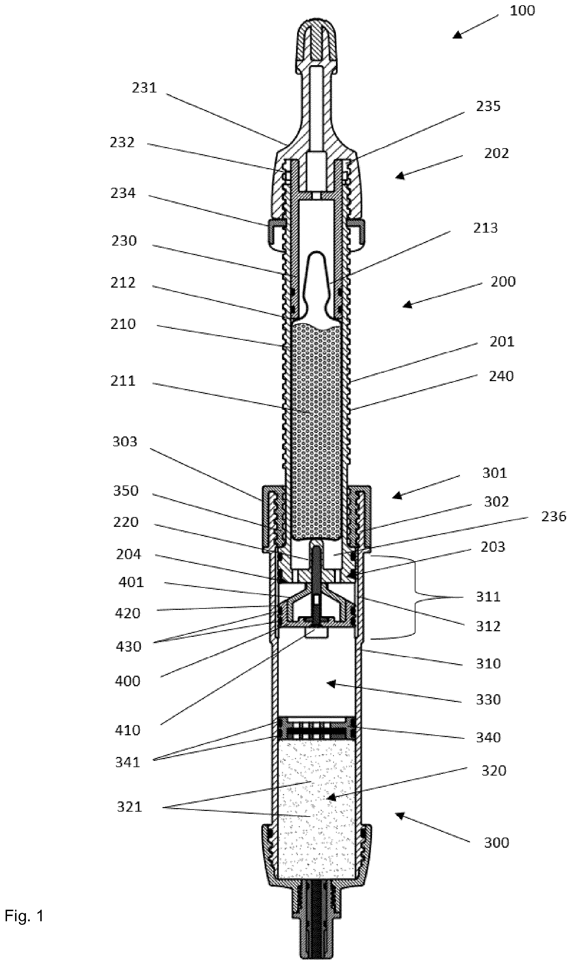

**Figur 1** zeigt eine Vorrichtung 100 in einem Ausgangszustand. Die Vorrichtung 100 ist einstückig, aber aus mehreren Bauteilen aufgebaut. Die Vorrichtung 100 weist ein erstes Behältnis 200 und ein zweites Behältnis 300 auf. Die Vorrichtung 100 ist rohrartig ausgestaltet. Das erste Behältnis 200 und das zweite Behältnis 300 sind axial miteinander verbunden. Das erste Behältnis 200 und das zweite Behältnis 300 sind gemeinsam auf der Mittelache der Vorrichtung 100 angeordnet. Das erste Behältnis 200 ragt in das zweite Behältnis 300 hinein.

Das erste Behältnis 200 dichtet das zweite Behältnis 300 zur Umgebung der Vorrichtung 100 ab.

Das erste Behältnis 200 besitzt auf einem Teil einer Außenfläche 201 ein Außengewinde 240. An einem ersten Ende 202 des ersten Behältnisses 200 ist ein Schraubmittel 231 angeordnet. Das Schraubmittel 231 umschließt das erste Ende 202 des ersten Behältnisses 200 haubenartig. Das Schraubmittel 231 hat ein Innengewinde 232, welches form- und/oder kraftschlüssig mit dem Außengewinde 240 zusammenwirkt. Das Schraubmittel 231 ist als Griff ausgestaltet. Als Griff ist der Krafteintrag zum gegenläufigen Drehen des ersten Behältnisses 200 gegen das zweite Behältnis 300 für den Anwender leichter aufzubringen. Das Innengewinde 232 ist nicht ganz auf das Außengewinde 240 aufgedreht, so dass ein Hohlraum A 235 zwischen Schraubmittel 231 und ersten Behältnis 200 besteht. An dem Schraubmittel 231 ist ein Förderkolben 230 in Form eines Schulterelements angeordnet. Der Förderkolben ist im Querschnitt H-förmig ausgestaltet. Der Förderkolben 230 ragt von dem ersten Ende 202 in das erste Behältnis 200 hinein. Der Förderkolben 230 in Form eines Schulterelements ist so ausgestaltet, dass ein Kontakt nur mit einer Schulter 212, nicht aber mit einem Kopf 213 eines Gefäßes 210 zustande kommen kann. Der Förderkolben 230 ist dabei rohrartig ausgeformt, wobei der Hohlraum einen derart großen Durchmesser aufweist, dass der Kopf 213 des Gefäßes 210, nicht aber die Schulter 212 des Gefäßes 210 aufgenommen werden kann. Somit schützt der Förderkolben 230 den Kopf 213 des Gefäßes 210 vor einem unkontrollierten Abbrechen. Der Förderkolben 230 kann somit nur auf die Schulter 212 des Gefäßes 210 einwirken. Das erste Behältnis 200 besitzt ein zweites Ende 203 mit einer Öffnungsvorrichtung 220 in Form eines Anstechdorns.

In dem ersten Behältnis 200 ist eine Monomerflüssigkeit 211 gelagert. Die Monomerflüssigkeit 211 ist dabei in dem Gefäß 210 in Form einer Glasampulle gelagert. Das Gefäß 210 ist bevorzugt als Glasampulle ausgeformt, da die Monomerflüssigkeit 211 in einer Glasampulle hermetisch dicht und steril gelagert werden kann. Das Gefäß 210 ist weiterhin bevorzugt als Glasampulle ausgeformt, da eine Glasampulle leicht geöffnet werden kann. Das Gefäß 210 ist zwischen der Öffnungsvorrichtung 220 und dem Förderkolben 230 derart gelagert, dass das Gefäß 210 sich nicht frei im ersten Behältnis 210 bewegen kann. Dadurch wird eine unkontrollierte Bewegung des Gefäßes 210 und damit ein versehentliches Zerbrechen oder Öffnen des Gefäßes verhindert. Das Gefäß 210, die Öffnungsvorrichtung 220 und das erste Behältnis 200 bilden einen Hohlraum B 236 aus.

An einer Außenseite des zweiten Endes 203 des ersten Behältnisses 200 ist ein Austragskolben 400 angebracht. Der Austragskolben 400 ist an einer dem ersten Behältnis zugewandten Oberseite 401 bombiert. Der Austragskolben 400 ist fest mit dem ersten Behältnis 200 verbunden. Der Austragkolben 400 ist form- und/oder kraftschlüssig mit dem ersten Behältnis 200 verbunden. Der Austragskolben ist mit einer Schraubvorrichtung 410 mit dem zweiten Ende 203 des ersten Gefäßes 200 verbunden. Der Austragskolben 400 und das erste Gefäß 200 sind einstückig ausgestaltet. Der Austragskolben 400 grenzt mit einer Außenfläche 420 an eine Behältniswand 310 des zweiten Behältnisses 300. Der Austragskolben weist an der Außenfläche 420 zwei Dichtungsringe 430 auf.

Das zweite Behältnis besitzt an einem ersten Ende 301 ein Innengewinde 302. Auf dem Innengewinde 302 ist eine Gewindehülse 303 mit einem Innengewinde 350 aufgebracht. Das Innengewinde 350 kann form- und/oder kraftschlüssig mit dem Außengewinde 240 zusammenwirken. Die Gewindehülse 303 bildet mit dem Innengewinde 202 und dem Außengewinde 350 eine Co-axiale doppelte Gewindepaarung. Durch eine Verwendung der Gewindehülse 303 kann das erste Behältnis 200 mit einem geringeren Durchmesser als ohne Verwendung der Gewindehülse 303 ausgebildet sein. Dies reduziert das Risiko einer Verkeilung zwischen ersten Behältnis 200 und zweitem Behältnis 300 bei der Verwendung der Vorrichtung 100. Die Gewindehülse 303 verhindert ein vollständiges Auseinanderziehen des ersten Behältnisses 200 aus dem zweiten Behältnis 300, da die Gewindehülse eine Hinterschneidung für das zweite Ende 203 des ersten Gefäßes 200 bildet. Das zweite Ende 203 des ersten Gefäßes 200 weist einen Außendurchmesser und die Gewindehülse 303 einen Innendurchmesser auf, wobei der Außendurchmesser des ersten Endes 203 größer als der Innendurchmesser der Gewindehülse 303 ist. Dies reduziert das Risiko eines versehentlichen Öffnens und damit einer Kontamination der Vorrichtung 100. Das zweite Behältnis 300 besitzt einen ersten Innenraum 320 und einen zweiten Innenraum 330. Im ersten Innenraum 320 ist ein Knochenzementpulver 321 gelagert. Der gesamte zweite Innenraum 330 ist mit dem Knochenzementpulver 321 ausgefüllt. Das Knochenzementpulver 321 ist so im ersten Innenraum 320 verdichtet, dass das Knochenzementpulvers 321 nicht frei beweglich ist. In den Zwischenräumen des Knochenzementpulvers 321 befindet sich ein Gas. Der erste Innenraum 320 ist mit dem zweiten Innenraum 330 über ein fluidleitendes Fördermittel 340 verbunden. Das Fördermittel 340 ist durchlässig für Gase und Flüssigkeiten, aber undurchlässig für Feststoffe, wie beispielsweise das Knochenzementpulver 321, ausgestaltet. Das Fördermittel 340 weist in Richtung der Behältniswand 310 zwei Dichtungsringe 341 auf.

Der zweite Innenraum 330 grenzt in einem oberen Bereich an den Austragskolben 400. Der Austragskolben 400 befindet sich in einer ersten Position auf Höhe eines ersten Teilstücks 311 des zweiten Behältnisses 300. Das erste Teilstück 311 besitzt mindestens eine Vertiefung 312 in der Behältniswand 310. Die Vertiefung 312 verläuft in axialer Richtung des zweiten Behältnisses 300. Die Vertiefung 312 hat eine längere axiale Ausdehnung als der Austragskolben 400. Die Vertiefung 312 ist derart ausgestaltet, dass die Monomerflüssigkeit 211 den Austragskolben 400 auf Höhe des ersten Teilstücks 311 umfließen und so in den zweiten Innenraum 330 gelangen kann.

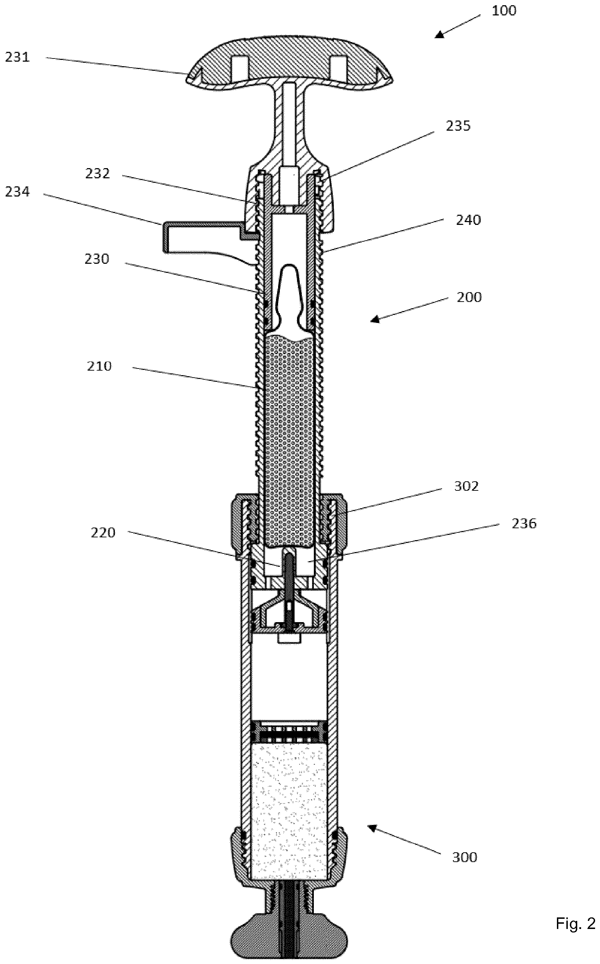

**Figur 2** zeigt die Vorrichtung 100 aus **Figur 1** um 90° nach rechts gedreht. Das Schraubmittel 231 ist als Griff für eine Hand ausgeformt. Um ein unabsichtliches Eindrehen des Förderkolbens 230 zu verhindern, ist direkt unterhalb des Schraubmittels 231 am Außengewinde 240 eine Sicherung 234 angebracht. Die Sicherung 234 blockiert das Außengewinde 240 für das Schraubmittel 231. Mit angelegter Sicherung 234 ist es nicht möglich, das Innengewinde 232 des Schraubmittels 231 vollständig auf das Außengewinde 240 zu drehen. Mit angelegter Sicherung 234 verbleibt der Hohlraum A 235 zwischen Schraubmittel 231 und ersten Behältnis 200 bestehen. Somit kann der Förderkolben 230 das Gefäß 210 nicht auf die Öffnungsvorrichtung 220 pressen. Mit angelegter Sicherung 234 verbleibt ein Hohlraum B 236 zwischen Öffnungsvorrichtung 220, Gefäß 210 und ersten Behältnis 200. Die Sicherung 234 lässt sich senkrecht zur Längsachse der Vorrichtung 100 abziehen. Die axiale Erstreckung des Hohlraums A 235 ist so lang, dass ein Herunterdrehen des Schraubmittels 231 auf das Außengewinde 240, und damit ein Fördern des Gefäßes 210 auf die Öffnungsvorrichtung 220, die strukturelle Integrität des Gefäßes 210 punktuell zerstört. Die axiale Erstreckung des Hohlraums A 235 ist kurz genug, so dass das Gefäß 210 nur punktuell aufgestochen, nicht aber vollständig zerstört wird. Eine axiale Erstreckung des Hohlraums B 236 ist vorteilhaft mindestens so lange wie eine axiale Erstreckung des Hohlraums A 235, da so durch ein Herunterdrehen des Schraubmittels 231 das Gefäß 210 nur punktuell geöffnet werden kann.

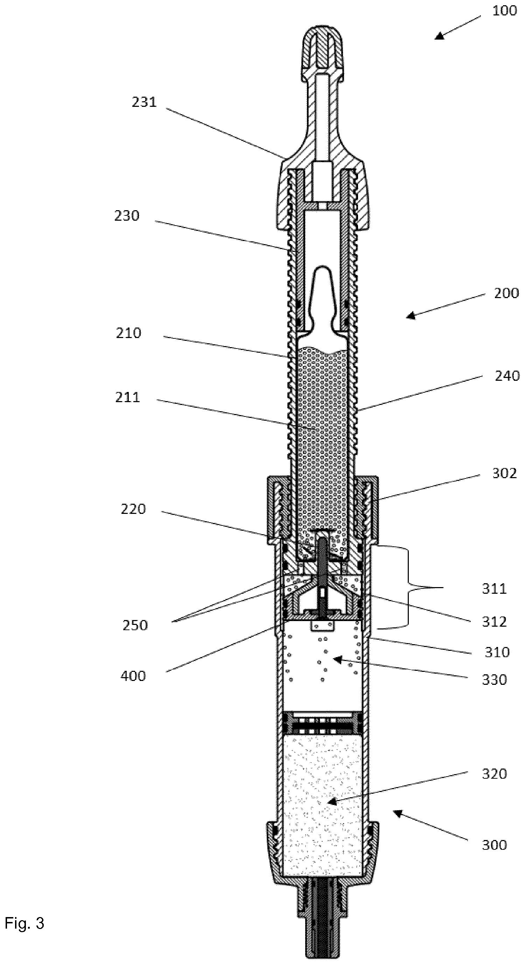

**Figur 3** zeigt, im Vergleich zu **Figur 2**, die Vorrichtung 100 nach Entfernen der Sicherung 234 (vgl. Figur 2) und mit heruntergedrehten Förderkolben 230. Das Schraubmittel 231 wurde vollständig auf das Außengewinde 240 aufgedreht. Das Schraubmittel 231 wurde derart auf das Außengewinde 240 aufgedreht, so dass kein Hohlraum A 235 (vgl. Figur 2) mehr zwischen Schraubmittel 231 und Außengewinde 240 besteht. Der Förderkolben 230 hat das Gefäß 210 in einer ersten Bewegung auf die Öffnungsvorrichtung 220 geschoben und damit geöffnet. Der Förderkolben hat das Gefäß 210 derart auf die Öffnungsvorrichtung 220 gepresst, dass kein Hohlraum B 236 (vgl. Figur 2) mehr zwischen ersten Behältnis 200, Öffnungsvorrichtung 220 und Gefäß 210 besteht. Die Öffnungsvorrichtung 220 hat die strukturelle Integrität des Gefäßes 210 punktuell zerstört. Nach erfolgtem Herunterdrehen des Schraubmittels 231 auf das Außengewinde 240, und damit dem Öffnen des Gefäßes 210, hat ein fortgeführtes gegenläufiges Drehen des ersten Behältnisses 200 gegen das zweite Behältnis 300 keinen Effekt auf die Vorrichtung 100. Dies dient der Reduzierung von Sicherheitsrisiken durch falsche Verwendung der Vorrichtung 100. Das Herunterdrehen des Schraubmittels 231 auf das Außengewinde 240 dient ausschließlich dem Öffnen des Gefäßes 210. Weiterführende Prozessschritte müssen separat eingeleitet werden. Dies verhindert, dass ein Anwender der Monomerflüssigkeit 211 nach dem Öffnen des Gefäßes 210 nicht genügende Zeit gibt, aus dem Gefäß 210 in den zweiten Innenraum 330 zu fließen. Der zweite Innenraum 330 ist räumlich über dem ersten Innenraum 320 angeordnet. Durch die Öffnung des Gefäßes 210 fließt die Monomerflüssigkeit 211 gemäß der Schwerkraft aus dem Gefäß 210. Die Monomerflüssigkeit 211 fließt gemäß der Schwerkraft durch wenigstens eine Durchführung 250 im Boden des ersten Gefäßes 200 in Richtung des Austragskolbens 400. Der Austragskolben befindet sich in der ersten Position auf Höhe des ersten Teilstücks 311. Die Monomerflüssigkeit 211 fließt um den Austragskolben 400 durch die Vertiefungen 312 in der Behältniswand 310 in den zweiten Innenraum 330. Die Monomerflüssigkeit 211 umströmt den Austragskolben 400 mittels der Vertiefungen 312 im ersten Teilstück 311. Der zweite Innenraum 330 umfasst ein Volumen, welches mindestens dem Volumen der Monomerflüssigkeit 211 entspricht. Der zweite Innenraum 330 kann die gesamte Monomerflüssigkeit 211 aufnehmen.

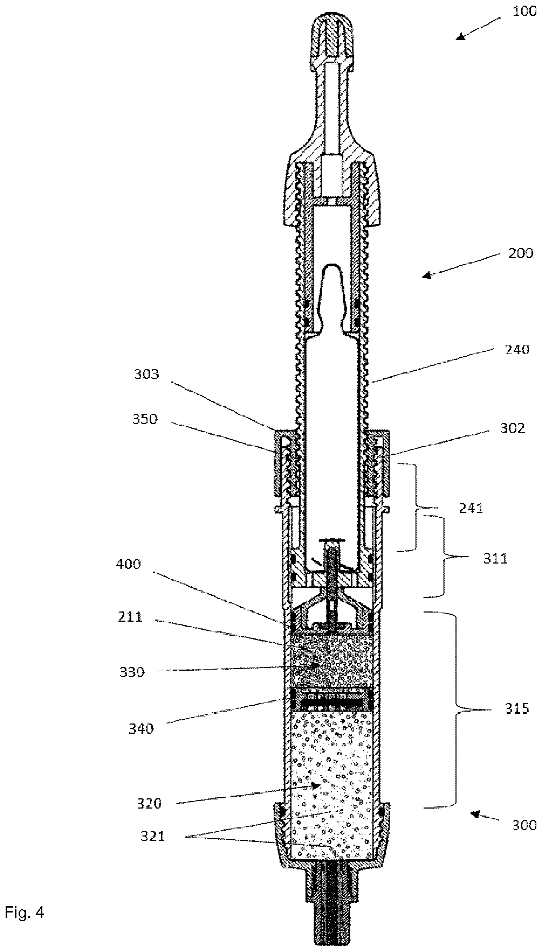

**Figur 4** zeigt, im Vergleich zu **Figur 3**, ein Ineinanderschieben mittels einer ersten axialen Verschiebung des ersten Behältnisses 200 in das zweite Behältnis 300. Im Laufe der ersten axialen Verschiebung wurde das erste Behältnis 200 in einem ersten Schritt in das zweite Behältnis 300 über eine Strecke entsprechend einer Länge eines Bereichs 241 des ersten Behältnisses 200 ohne Außengewinde heruntergedrückt. Die Länge des Bereichs 241 ist so lang, dass der Austragskolben 400 aus der Höhe des ersten Teilstücks 311 auf die Höhe eines zweiten Teilstücks 315 verschoben wird. Das zweite Teilstück 315 weist keine Vertiefungen in der Behältniswand 310 auf. Der Austragskolben 400 trennt auf Höhe des zweiten Teilstücks 311 den zweiten Innenraum 330 fluidundurchlässig in Richtung des ersten Behältnisses 200 ab. Der erste Schritt dient dazu, die Monomerflüssigkeit 211 dicht im zweiten Innenraum 330 gegenüber dem ersten Behältnis 200 abzutrennen. Der erste Schritt der ersten axialen Verschiebung dient somit der Reduzierung von Sicherheitsrisiken durch falsche Verwendung der Vorrichtung 100. Der erste Schritt der ersten axialen Verschiebung stellt sicher, dass die gesamte in den zweiten Innenraum 330 geflossene Monomerflüssigkeit 211 für weitere Prozessschritte zur Verfügung steht und nicht wieder zurück in das erste Behältnis 200 fließen kann.

Nach Beendigung des ersten Schritts wird die Vorrichtung 100 so gedreht, dass der erste Innenraum 320 räumlich über dem zweiten Innenraum 330 angeordnet ist. Dies hat den Vorteil, dass eine folgende Förderung der Monomerflüssigkeit 211 in den ersten Innenraum 320 von räumlich unten nach oben verläuft. Somit kann ein Gas, welches zwischen den einzelnen Partikeln des Knochenzementpulvers 321 vorhanden ist, beim Fördern der Monomerflüssigkeit 211 in den ersten Innenraum 320 nach oben ausweichen, was das Risiko von Lufteinschlüssen im Knochenzement reduziert.

In einem zweiten Schritt der ersten axialen Verschiebung wirkt das Außengewinde 240 des ersten Behältnisses 200 durch eine Drehung um die Längsachse der Vorrichtung 100 form- und/oder kraftschlüssig mit dem Innengewinde 302 des zweiten Behältnisses 300 zusammen. Dabei kann das Zusammenwirken entweder durch einen direkten Kontakt des Innengewindes 302 mit dem Außengewinde 240, oder über eine Einwirkung der Gewindehülse 303 erfolgen. Gezeigt ist in der Figur das Zusammenwirken über die Gewindehülse 303. Die erste axiale Verschiebung wird im Folgenden über eine gegenläufige Drehung des ersten Behältnisses 200 relativ zum zweiten Behältnis 300 fortgeführt. Die Vorrichtung 100 ist derart ausgestaltet, dass die gegenläufige Drehung des ersten Behältnisses relativ zum zweiten Behältnis 300 nur in Richtung der ersten axialen Verschiebung stattfinden kann. Die Vorrichtung 100 ist derart ausgestaltet, dass nach Einsetzten des zweiten Schrittes der ersten axialen Verschiebung, das erste Behältnis 200 und das zweite Behältnis 300 nicht wieder auseinander gedreht werden können. Dies hat einerseits den Vorteil, dass ein Anwender keine zusätzliche Kraft aufwenden muss, um ein Zurückdrehen der Vorrichtung 100 zu verhindern. Zum anderen wird ein Risiko der Bildung von Luftblasen reduziert.

Durch die erste axiale Verschiebung des ersten Behältnisses 200 in das zweite Behältnis 300 ist der Austragskolben 400 aus dem ersten Teilstück 311 in ein zweites Teilstück 315 verschoben worden und eine Förderung der Monomerflüssigkeit 211 in den ersten Innenraum 320 hat eingesetzt. Durch eine fortgesetzte erste axiale Verschiebung des ersten Behältnisses 200 in das zweite Behältnis 300 wird die Monomerflüssigkeit aus dem zweiten Innenraum 330 über das Fördermittel 340 in das Knochenzementpulver 321 im ersten Innenraum 320 gepresst.

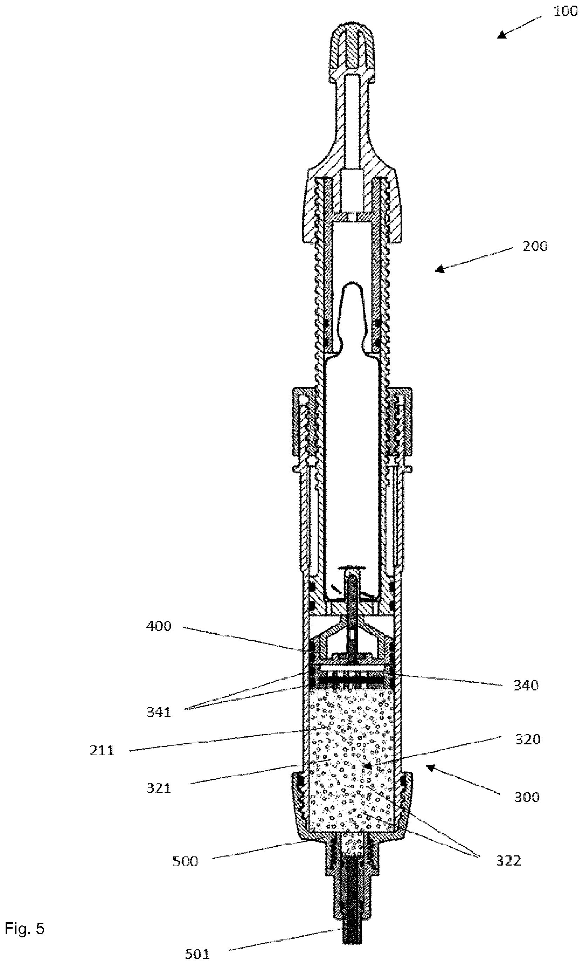

**Figur 5** zeigt die Vorrichtung 100 nach Beendigung der ersten axialen Verschiebung. Die erste axiale Verschiebung ist beendet, wenn der Austragskolben 400 das Fördermittel 340 erreicht. Das Erreichen des Fördermittels 340 wird einem Anwender der Vorrichtung 100 durch einen erhöhten Gegendruck im Laufe des gegenläufigen Drehens des ersten Behältnisses 200 gegen das zweite Behältnis 300 angezeigt. Einerseits ist das Fördermittel 340 derart fest im zweiten Behältnis 300 arretiert, dass das Knochenzementpulver 321 verdichtet im ersten Innenraum 320 lagerbar ist und ein Anwender einen merkbaren Gegendruck bei Vollendung der ersten axialen Verschiebung verspürt. Andererseits ist das Fördermittel 340 leicht genug im zweiten Behältnis 300 verschiebbar, so dass ein Anwender keine Schwierigkeiten beim Verschieben des Fördermittels 340 bei der weiteren Verwendung der Vorrichtung 100 hat. Das Fördermittel 340 weist auf einer Außenfläche zwei Dichtungsringe 341 auf. Die Dichtungsringe 341 gewährleisten einen Kompromiss zwischen fester Arretierung und leichter Verschiebbarkeit des Fördermittels 340. Die gesamte Monomerflüssigkeit 211 ist in den ersten Innenraum 320 gefördert worden. Die Monomerflüssigkeit 211 hat das Gas in den Zwischenräumen des Knochenzementpulvers 321 verdrängt. Das Gas in den Zwischenräumen des Knochenzementpulvers 321 ist in Richtung einer Austragsöffnung 500, welche angrenzend an den ersten Innenraum 320 angeordnet ist, gedrängt. Die Austragsöffnung 500 ist mit einem Verschlussstift 501 versehen. Der Verschlussstift 501 verhindert ein ungewolltes Austragen des Knochenzementpulvers 321 aus der Vorrichtung 100. Der Verschlussstift 501 ist für Gase und optional auch für die Monomerflüssigkeit 211 durchlässig ausgestaltet. Das Gas aus den Zwischenräumen des Knochenzementpulvers 321 ist durch den Verschlussstift 501 aus der Vorrichtung 100 ausgetrieben worden.

Nach Beendigung der ersten axialen Verschiebung ist das gesamte Knochenzementpulver 321 von der Monomerflüssigkeit 211 benetzt. Nach vollständiger Benetzung des Knochenzementpulvers 321 durch die Monomerflüssigkeit 211 dehnt sich das Volumen des Knochenzementpulvers 321 aus. Bei der Ausdehnung des Knochenzementpulvers 321 wird der Verschlussstift 501 teilweise aus der Austragsöffnung 500 ausgetrieben. Dies zeigt dem Anwender eine ordnungsgemäße Funktion der Vorrichtung 100 und eine vollständige Benetzung des Knochenzementpulvers 321 mit der Monomerflüssigkeit 211 an. Durch die Vermischung von Knochenzementpulver 321 und Monomerflüssigkeit 211 bildet sich ein Knochenzement 322 aus. Das Zusammenführen von Knochenzementpulver 321 und Monomerflüssigkeit 211 resultiert in einer Quellung des Knochenzementpulvers 321.

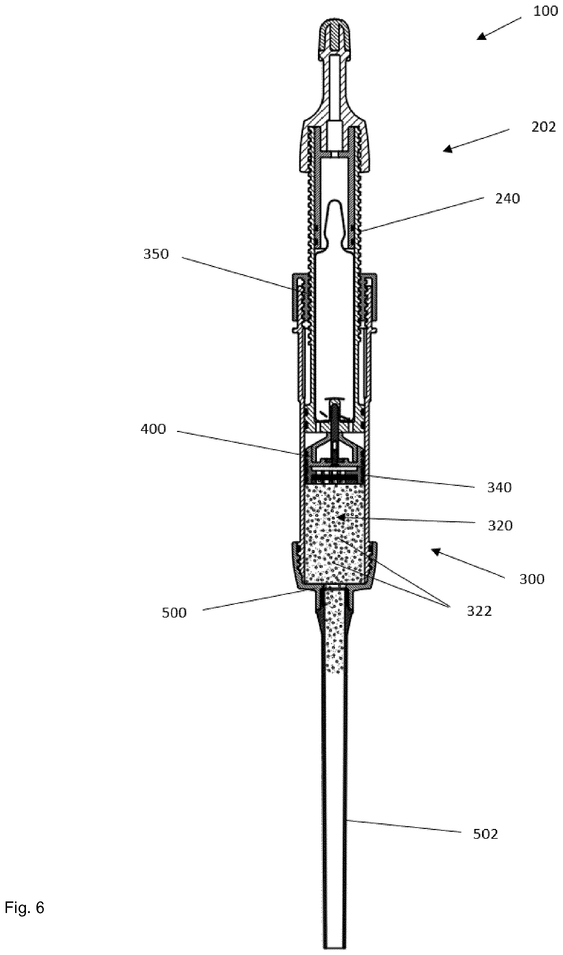

**Figur 6** zeigt die Vorrichtung 100 im Verlauf einer zweiten axialen Verschiebung des ersten Behältnisses 200 in das zweite Behältnis 300. Der Verschlussstift 501 (vgl. Figur 5) wurde durch eine Austragskanüle 502 ersetzt. Die zweite axiale Verschiebung wird durch eine fortgesetzte gegenläufige Drehung des ersten Behältnisses 200 gegen das zweite Behältnis 300 um die Längsachse der Vorrichtung 100 durchgeführt, wobei das Innengewinde 350 der Gewindehülse 303 form- und/oder kraftschlüssig mit dem Außengewinde 240 zusammenwirkt. Im Laufe der zweiten axialen Verschiebung schiebt der Austragskolben 400 das Fördermittel 340 in Richtung Austragsöffnung 500. Durch die zweite axiale Verschiebung des ersten Behältnisses 200 in das zweite Behältnis 300 wird der Knochenzement 322 aus dem zweiten Innenraum 320 aus der Vorrichtung 100 in die Austragkanüle 502 gefördert.

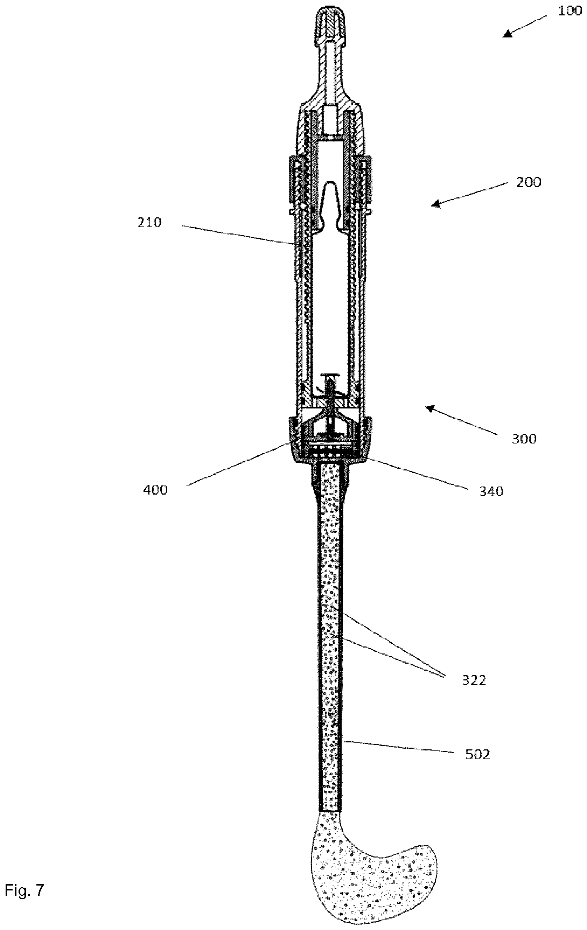

**Figur 7** zeigt die Vorrichtung 100 nach Beendigung der zweiten axialen Verschiebung des ersten Behältnisses 200 in das zweite Behältnis 300. Das Fördermittel 340 ist durch den Austragskolben 400 bis an den Boden des zweiten Behältnisses 300 geschoben worden. Der Knochenzement 322 ist vollständig aus der Vorrichtung 100 ausgepresst worden. Die Austragskanüle 502 sorgt für die Applikation des Knochenzements 322 an gewünschter Stelle. Die Austragskanüle 502 ist noch mit Resten des Knochenzements 322 gefüllt. Die Vorrichtung 100 hat durch das Ineinanderschieben des ersten Behältnisses 200 in das zweite Behältnis 300 eine kürzere axiale Ausdehnung angenommen. Das Gefäß 210 ist nach Beendigung des Austragens des Knochenzements 322 nicht vollständig zerstört. Das Gefäß 210 ist nach Beendigung des Austragens des Knochenzements 322 durch die Öffnungsvorrichtung 220 nur punktuell geöffnet.

**Figur 1** bis **Figur 7** zeigen die Vorrichtung 100 im Zuge des Bereitstellens und Mischens der Ausgangskomponenten sowie des Austragens des Knochenzements 322. Ein Anwender beginnt mit einem Entfernen der Sicherung 234 vom Außengewinde 240 des ersten Behältnisses 200. Die Sicherung 234 hat den Zweck, ein ungewolltes Öffnen des Gefäßes 210 mit der Monomerflüssigkeit 211 zu verhindern. Ist die Sicherung 234 entfernt, schraubt der Anwender das anfänglich nicht vollständig auf das Außengewinde 240 des ersten Behältnisses 200 aufgedrehte Schraubmittel 231 herunter. Das Schraubmittel 231 wirkt dabei auf den Förderkolben 230 ein, so dass dieser weiter in das erste Behältnis 200 eingeschoben wird. Insofern muss der Förderkolben 230 axial beweglich im ersten Behältnis 200 angeordnet sein. Das Schrauben des Schaubmittels 231 resultiert in einer axialen Bewegung des Förderkolbens 230 hin zum Gefäß 210. Dabei überträgt der Förderkolben 230 - im Wesentlichen - die axiale Bewegung des Schraubmittels 231 auf die Schulter des Gefäßes 210, um dieses auf die Öffnungsvorrichtung 220 zu pressen und damit punktuell zu öffnen. Der Förderkolben 230 dient als ein Übertragungsmittel der im Gewinde, beim Aufschrauben des Schraubmittels 231 auf das Außengewinde 240, entstehenden, in die Vorrichtung 100 gerichteten Kraft auf das Gefäß 210.

Die Schulter 212 des Gefäßes 210 ist strukturell so stabil, dass das Öffnen des Gefäßes 210 nur angrenzend an die Öffnungsvorrichtung 220 geschieht. In einer Variante lässt sich das Schraubmittel 231 nur so weit auf das Außengewinde 240 herunterdrehen, dass das Gefäß 210 punktuell, aber nicht vollständig zerstört wird. Nach vollständigem Aufdrehen des Schraubmittels 231 auf das Außengewinde 240 führt eine fortgeführte Drehbewegung zu keiner weiteren axialen Bewegung des Förderkolbens 230. Lediglich das erste Behältnis 200 kann dann um seine Längsachse innerhalb der Vorrichtung 100 gedreht werden. Ist das Gefäß 210 geöffnet, fließt die Monomerflüssigkeit 211 aus dem Inneren des Gefäßes 210 in Richtung des Austragskolbens 400. Wie oben beschrieben, ist der Austragskolben 400 in der ersten Position für die Monomerflüssigkeit 211 durchlässig. Um die Monomerflüssigkeit 211 in den zweiten Innenraum 330 zu fördern, wird die Vorrichtung 100 so gehalten, dass das Behältnis 200 räumlich über dem zweiten Behältnis 300 liegt. Dadurch wird die Schwerkraft zum Fördern der Monomerflüssigkeit 211 in den zweiten Innenraum 330 ausgenutzt, weshalb keine externe Apparatur, wie beispielsweise eine Pumpe oder ein Vakuumanschluss, notwendig ist. Um ein im Wesentlichen vollständiges Überführen der Monomerflüssigkeit 211 in den zweiten Innenraum 330 zu gewährleisten, kann die Vorrichtung 100 in der oben beschrieben Haltung, mit dem zweiten Behältnis 300 nach unten, eine Zeitspanne von 60 Sekunden, insbesondere 30 Sekunden, bevorzugt 10 Sekunden gehalten werden. Um nach dem Fördern der Monomerflüssigkeit 211 in den zweiten Innenraum 330 ein Rückfließen in das erste Behältnis 200 zu verhindern, wird das erste Behältnis 200 mittels der ersten axialen Bewegung zumindest teilweise in das zweite Behältnis 300 eingeschoben. Dies überführt den Austragskolben 400 aus der ersten Position in die zweite Position. Somit kann die Monomerflüssigkeit 211 nicht mehr durch die Vertiefung 312 in Richtung des ersten Behältnisses 200 rückfließen.

Im Anschluss wird die Vorrichtung 100 so gedreht, dass das zweite Behältnis 300 räumlich über dem ersten Behältnis 200 angeordnet ist. Dies hat den Vorteil, dass beim anschließenden Fördern der Monomerflüssigkeit 211 aus dem zweiten Innenraum 330 in den ersten Innenraum 320 das Gas, welches sich im zweiten Innenraum 330 und zwischen den Partikeln des Zementpulvers 321 befindet, in Richtung der Austragsöffnung 500 verdrängt wird. Dieses gezielte Leiten des Gases in Richtung der Austragsöffnung 500 der Vorrichtung 100 reduziert das Risiko von Gaseinschlüssen innerhalb des gemischten Knochenzements 322.

Um die Monomerflüssigkeit 211 aus dem zweiten Innenraum 330 in den ersten Innenraum 311 zu fördern, bedarf es einer Krafteinwirkung auf den Austragskolben 400 in Richtung des Fördermittels 340. Dazu wirkt das Außengewinde 240 des ersten Behältnisses 200 mit dem Innengewinde 302 des zweiten Behältnisses 300 zusammen. Das Zusammenwirken kann direkt - also form- und/oder kraftschlüssig - geschehen, oder, wie gezeigt, durch Mitwirkung einer Gewindehülse 303. Der Anwender dreht dafür das Schraubmittel 231 derart, dass das Außengewinde 240 in das Innengewinde 350 der Gewindehülse 303 eingreift und das erste Behältnis 200 über diese Drehbewegung in das zweite Behältnis 300 eingeschraubt wird. Vorzugsweise lässt sich das Einschrauben nicht rückgängig machen, wodurch wiederum eine Entstehung von Gaseinschlüssen reduziert wird. Eine entsprechende Rückschraubsperre sorgt dafür, dass bei einem Unterbrechen des Förderns etwaige in der Vorrichtung 100 aufgebaute Kräfte nicht zu einer Vergrößerung eines Abstandes zwischen Austragskolben 400 und des Fördermittels 340, insbesondere Austragsöffnung 500 sorgen.

Die erste axiale Verschiebung ist abgeschlossen, wenn der Austragskolben 400 das Fördermittel 340 erreicht hat, insbesondere berührt. Zu diesem Zeitpunkt ist das Fördern der Monomerflüssigkeit 211 in den ersten Innenraum 320 beendet. Dem Anwender wird die Beendigung der ersten axialen Verschiebung durch einen erhöhten Gegendruck beim Drehen des Schraubmittels 231 und durch das teilweise Austreiben des Verschlussstifts 502 angezeigt.

Bevor der Anwender über ein fortgeführtes Drehen des Schraubmittels 231 die zweite axiale Verschiebung einleitet, verbleibt die Vorrichtung in der beschriebenen Position, um einem Ausbilden des Knochenzements 322 genügend Zeit zu gewährleisten. Die Zeitspanne hängt von der Zusammensetzung und den Eigenschaften der Ausgangsmaterialien ab und kann eine Dauer von 5 Sekunden bis 5 Minuten einnehmen. Im Anschluss wird die zweite axiale Verschiebung des ersten Behältnisses 200 in das zweite Behältnis 300 eingeleitet. Je nach Applikationsort des Knochenzements 322 ist es sinnvoll, die Vorrichtung 100 an der Austragsöffnung 500 vor dem Austragen des Knochenzements 322 mit der Austragkanüle 502 auszustatten. Die zweite axiale Verschiebung wird so lange fortgesetzt, bis die gewünschte Menge oder der gesamte Knochenzement 322 aus der Vorrichtung 100 ausgetragen wurde. Die räumliche Ausrichtung der Vorrichtung 100 im Zuge der zweiten axialen Verschiebung kann beliebig, je nach Bedarf, gewählt werden.

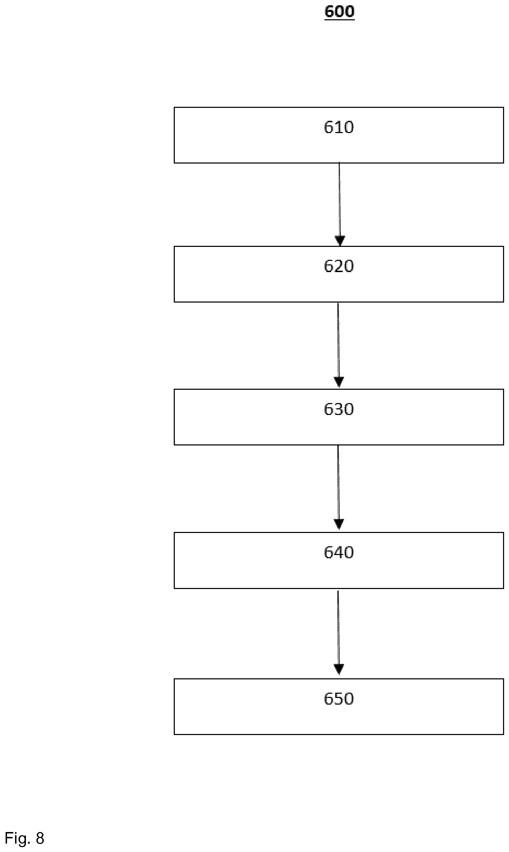

**Figur 8** zeigt ein Flussdiagramm enthaltend die Schritte 610 bis 650 eines Verfahrens 600 zur Bereitstellung eines Knochenzements aus zwei Ausgangskomponenten mittels der Vorrichtung 100 umfassend das hohlzylinderförmiges erstes Behältnis 200, in dem die Monomerflüssigkeit 211 als erste Ausgangskomponente in dem Gefäß 210 lagert, das hohlzylinderförmige zweite Behältnis 300 aufweisend die Behältniswand 310, den ersten Innenraum 320 und den zweiten Innenraum 330, wobei in dem ersten Innenraum 320 das Knochenzementpulver 321 als zweite Ausgangskomponente lagert, und wobei die Monomerflüssigkeit 211 in den zweiten Innenraum 330 förderbar ist, das fluidleitendes Fördermittel 340, angeordnet zwischen dem ersten Innenraum 320 und dem zweiten Innenraum 330, wobei das erste Behältnis 200 und das zweite Behältnis 300 axial miteinander verbunden sind. In einer bevorzugten Ausführungsform des Verfahrens 600 ist das Gefäß 210 aufgrund der leichten Sterilisierbarkeit und guten Zerstörbarkeit eine Glasampulle. In einer weiteren bevorzugten Ausführungsform des Verfahrens 600 befindet sich der Austragskolben 400 auf Höhe einer ersten Position innerhalb des zweiten Gefäßes 300. Die erste Position ist dadurch gekennzeichnet, dass die an den Austragskolben 400 grenzende Behältniswand 310 Vertiefungen 312 aufweist.

In einem ersten Schritt 610 wird das Gefäß 210 geöffnet. Bevorzugt wird das Gefäß 210 nur punktuell geöffnet und nicht vollständig zerstört. In einer bevorzugten Ausführungsform des Verfahrens 600 ist das erste Behältnis 200 vor dem Schritt 610 räumlich über dem zweiten Behältnis 300 angeordnet, so dass die Monomerflüssigkeit 211 gemäß der Schwerkraft nach unten in Richtung des zweiten Behältnisses 300 fließen kann. In einer weiteren bevorzugten Ausführungsform des Verfahrens 600 erfolgt Schritt 610 durch ein Herunterdrehen des Förderkolbens 230, welcher das Gefäß 210 auf die Öffnungsvorrichtung 220 in Form eines Anstechdorns presst und damit punktuell öffnet.

In einem zweiten Schritt 620 fließt die Monomerflüssigkeit 211 aus dem Gefäß 210 um den Austragskolben 400 herum in den zweiten Innenraum 330. In einer weiteren bevorzugten Ausführungsform des Schrittes 620 umströmt die Monomerflüssigkeit 211 den Austragskolben 400 über mindestens eine Vertiefung 312 in der Behältniswand 310. In einer weiteren bevorzugten Ausführungsform des Schritts 620 umströmt die Monomerflüssigkeit 211 den Austragskolben 400 über Vertiefungen 312 in Form einer Nut. In einer bevorzugten Ausführungsform des Schritts 620 ist der Austragskolben 400 bombiert, damit die gesamte Monomerflüssigkeit 211 in Richtung der Vertiefungen 312 geleitet wird.

In einem dritten Schritt 630 wird die Monomerflüssigkeit 211 aus dem zweiten Innenraum 330 in den ersten Innenraum 320 durch eine erste Verschiebung des ersten Behältnisses 200 in das zweite Behältnis 300 gefördert. In einer bevorzugten Ausführungsform des Schritts 630 erfolgt in einem ersten Schritt ein Einschieben des Austragskolbens 400 aus der ersten Position in eine zweite Position. Die zweite Position ist dadurch gekennzeichnet, dass die an den Austragskolben 400 grenzende Behältniswand 310 vertiefungsfrei ausgestaltet ist. Die Monomerflüssigkeit 211 kann den Austragskolben 400 auf Höhe der zweiten Position nicht umströmen. Der Austragskolben 400 schließt den zweiten Innenraum 330 auf Höhe der zweiten Position für Flüssigkeiten und Feststoffe gegenüber dem ersten Behältnis 200 ab. In einer weiteren bevorzugten Ausführungsform des Schritts 630 wird die Vorrichtung 100 nach dem ersten Schritt so gedreht, dass das zweite Behältnis 300 räumlich über dem ersten Behältnis 200 angeordnet ist. Dies hat zur Folge, dass ein zwischen den Partikeln des Knochenzementpulvers 321 befindliches Gas sich im weiteren Verlauf des Verfahrens 600 störungsfreier, mit reduziertem Risiko von Lufteinschlüssen, aus dem Knochenzementpulver 321 durch die Monomerflüssigkeit 211 verdrängen lässt. In einer weiteren bevorzugten Ausführungsform des Schrittes 630 weist das erste Behältnis 200 ein Außengewinde 240 und das zweite Behältnis 300 ein Innengewinde 302 auf, wobei Außengewinde 240 und Innengewinde 302 form- und/oder kraftschlüssig zusammenwirkten können um das erste Behältnis 200 in das zweite Behältnis 300 zu verschieben. In einer weiteren bevorzugten Ausführungsform des Schrittes 630 weist die Vorrichtung eine Gewindehülse 303 auf. Die Gewindehülse bildet mit dem Innengewinde 302 und dem Außengewinde 240 eine Co-axiale doppelte Gewindepaarung. In einem zweiten Schritt der ersten axialen Verschiebung wirkt das Außengewinde 240 und das Innengewinde 302, bevorzugt über die Gewindehülse 303, derart miteinander, dass das erste Behältnis 200 in das zweite Behältnis 300 eingeschoben wird. Der zweite Schritt der ersten axialen Verschiebung fördert die Monomerflüssigkeit 211 aus dem zweiten Innenraum 330 in den ersten Innenraum 320.

In einem vierten Schritt 640 kommt es zu einer Ausbildung des Knochenzements 322 aus Knochenzementpulver 321 und Monomerflüssigkeit 211. In einer bevorzugten Ausführungsform des Schritts 640 startet die Ausbildung des Knochenzements 322 mit einer Benetzung des Knochenzementpulvers 321 durch die Monomerflüssigkeit 211. Nach der Benetzung kommt es zu einem Anquellen des Knochenzementpulvers 321.

In einem fünften Schritt 650 wird durch eine zweite Verschiebung des ersten Behältnisses 200 in das zweite Behältnis 300 der Knochenzement 322 aus der Vorrichtung 100 ausgetrieben. In einer bevorzugten Ausführungsform des Schritts 650 erfolgt das Austreiben des Knochenzements 322 aus der Vorrichtung 100 über eine fortgeführte gegenläufige Drehung des ersten Behältnisses 200 gegen das zweite Behältnis 300. In einer weiteren bevorzugten Ausführungsform ist das Gefäß 210 nach Austragen des Knochenzements 322 nicht vollständig zerstört. In einer weiteren bevorzugten Ausführungsform des Verfahrens 600 ist das Gefäß 210 nach Austragen des Knochenzements 322 nur punktuell geöffnet.

Ein Vorteil des erfindungsgemäßen Verfahrens 600 ist, dass das Gefäß 210 im Zuge des Mischens und Austragens des Knochenzements 322 nicht vollständig zerstört werden muss. Dies verringert einerseits den benötigten Kraftaufwand beim Anwender, andererseits werden die oben beschriebenen Nachteile von Bruchstücken des Gefäßes 210 vermieden. Ein anderer Vorteil des erfindungsgemäßen Verfahrens 600 ist, dass die Teilschritte 630 und 650 sequentiell mit gleichartigen Verfahrensschritten ablaufen und keine Möglichkeit besteht, die Reihenfolge der Verfahrensschritte zu ändern oder zu verwechseln. Somit ist das Verfahren 600 einfach, sicher und schnell in der Ausführung.

Zusammenfassend lässt sich feststellen, dass die Erfindung gekennzeichnet ist durch eine Vorrichtung, die zwei Behältnisse aufweist, wobei im ersten Behältnis die Monomerflüssigkeit lagert und im zweiten Behältnis das Knochenzementpulver. Erfindungsgemäß wird das erste Behältnis in das zweite Behältnis geschoben, wobei das axiale Einschieben des ersten Behältnisses sequentiell nacheinander
1. das Gefäß für die Monomerflüssigkeit öffnet,
2. die Monomerflüssigkeit in das Knochenzementpulver fördert und
3. den fertigen, mischungsfrei aus Monomerflüssigkeit und Knochenzementpulver gebildeten, Knochenzement austrägt.

Während die Erfindung in den Zeichnungen und der vorstehenden Beschreibung ausführlich dargestellt und beschrieben wurde, sind diese Abbildungen und Beschreibungen als illustrativ oder exemplarisch und keinesfalls als restriktiv anzusehen. Die Erfindung ist nicht auf die offenbarten Ausführungsformen beschränkt. Variationen der offenbarten Ausführungsformen können von Fachleuten aus einem Studium der Zeichnungen, der Offenbarung und den abhängigen Ansprüchen verstanden und ausgeführt werden.

In den Ansprüchen schließt das Wort "umfassend" andere Element oder Schritte nicht aus. Der unbestimmte Artikel "ein" oder "eine" schließt ebenso eine Vielzahl nicht aus. Die bloße Tatsache, dass bestimmte Merkmale in voneinander unabhängigen Ansprüchen zitiert werden, deutet nicht darauf hin, dass eine Kombination dieser Merkmale nicht vorteilhaft genutzt werden kann. Etwaige Bezugszeichen in den Ansprüchen sollten nicht als Einschränkungen ausgelegt werden.

### Bezugszeichenliste

- 100: Vorrichtung
- 200: erstes Behältnis
- 201: Außenfläche des ersten Behältnisses
- 202: erstes Ende des ersten Behältnisses
- 203: zweites Ende des ersten Behältnisses
- 210: Gefäß
- 211: Monomerflüssigkeit
- 212: Schulter des Gefäßes
- 213: Kopf des Gefäßes
- 220: Öffnungsvorrichtung
- 230: Förderkolben
- 231: Schraubmittel
- 232: Innengewinde des Schraubmittels
- 233: Schulterelement
- 234: Sicherung des Förderkolbens
- 235: Hohlraum A
- 236: Hohlraum B
- 240: Außengewinde
- 241: Bereich ohne Außengewinde
- 250: Durchführungen
- 300: zweites Behältnis
- 301: erstes Ende des zweiten Behältnisses
- 302: Innengewinde des zweiten Behältnisses
- 303: Gewindehülse
- 310: Behältniswand
- 311: erstes Teilstück
- 312: Vertiefung
- 315: zweites Teilstück
- 320: erster Innenraum
- 321: Knochenzementpulver
- 322: Knochenzement
- 330: zweiter Innenraum
- 340: Fördermittel
- 341: Dichtungsringe des Fördermittels
- 350: Innengewinde der Gewindehülse
- 400: Austragskolben
- 401: Oberseite Austragskolben
- 410: Schraubvorrichtung
- 420: Außenfläche des Austragskolbens
- 430: Dichtungsringe des Austragskolbens
- 500: Austragsöffnung
- 501: Verschlussstift
- 502: Austragskanüle
- 600: Verfahren zur Bereitstellung eines Knochenzements
- 610: Öffnen des Gefäßes
- 620: Fließen der Monomerflüssigkeit
- 630: erste Verschiebung des ersten Behältnisses in das zweite Behältnis
- 640: Ausbildung des Knochenzements
- 650: zweite Verschiebung des ersten Behältnisses in das zweite Behältnis

## Patentansprüche

1. Verfahren (600) zur Herstellung eines Knochenzements (322) aus wenigstens zwei Ausgangskomponenten in einer Vorrichtung (100) aufweisend
ein erstes hohlzylinderförmiges Behältnis (200) und ein zweites hohlzylinderförmiges Behältnis (300), wobei das zweite Behältnis einen ersten Innenraum (320) und einen zweiten Innenraum (330) aufweist,
ein in dem ersten Behältnis (200) angeordnetes Gefäß (210), wobei das Gefäß (210) eine Monomerflüssigkeit (211) lagert,
einen Austragskolben (400), angeordnet zwischen erstem Behältnis (200) und zweitem Innenraum (330) umfassend **die Schritte:**
a. Öffnen (610) des Gefäßes (210) mittels einer ersten Bewegung des Gefäßes (210) relativ zum zweiten Behältnis (300),
b. Fließen der Monomerflüssigkeit (211) in den zweiten Innenraum (330), während der Austragskolben (400) für Gase und Flüssigkeiten durchlässig ausgestaltet ist,
c. Fördern der Monomerflüssigkeit (211) in ein, im ersten Innenraum lagerndes Knochenzementpulver (321) mittels einer ersten axial Verschiebung (630) des ersten Behältnisses (200) in das zweite Behältnis (300) hinein, wobei der Austragskolben (400) zum Fördern der Monomerflüssigkeit (211) eine Transformation von für Gase und Flüssigkeiten durchlässig zu für Flüssigkeiten und Feststoffe undurchlässig durchläuft, und
d. Auspressen des, aus der Monomerflüssigkeit (211) und dem Knochenzementpulver (321) gebildeten Knochenzements (322) aus der Vorrichtung (100) mittels einer zweiten axialen Verschiebung (650) des ersten Behältnisses (200) in das zweite Behältnis (300) hinein.

2. Verfahren (600) nach Anspruch 1, **dadurch gekennzeichnet, dass** zum Öffnen (610) gemäß Schritt a) das Gefäß (210) auf eine im ersten Behältnis (200) angeordnete Öffnungsvorrichtung (220) gepresst wird.

3. Verfahren (600) nach Anspruch 2, **dadurch gekennzeichnet, dass** zum Öffnen (610) gemäß Schritt a) das Gefäß (210) mittels eines im ersten Behältnis (200) angeordneten Förderkolbens (230) auf die Öffnungsvorrichtung (220) gepresst wird.

4. Verfahren (600) nach einem der vorhergehenden Ansprüche, **dadurch gekennzeichnet, dass** an dem ersten Behältnis (200) ein Schraubmittel (231) angeordnet ist, welches kraft- und/oder formschlüssig auf den Förderkolben (230) wirkt, um das Gefäß (210) in der ersten Bewegung auf die Öffnungsvorrichtung (220) zu pressen.

5. Verfahren (600) nach einem der vorhergehenden Ansprüche, **dadurch gekennzeichnet, dass** das erste Behältnis (200) einen Austragskolben (400) aufweist, welcher bei der ersten Verschiebung (630) in den zweiten Innenraum (330) bewegt wird.

6. Verfahren (600) nach einem der vorhergehenden Ansprüche, **dadurch gekennzeichnet, dass** der Austragskolben (400) bei der ersten Verschiebung (630) die Monomerflüssigkeit (211) in das Knochenzementpulver (321) fördert und bei der zweiten Verschiebung (650) den Knochenzement (322) aus der Vorrichtung auspresst.

7. Vorrichtung (100) zum Bereitstellen eines Knochenzements (322) aus zwei Ausgangskomponenten, aufweisend
ein hohlzylinderförmiges erstes Behältnis (200), in dem ein Gefäß (210) für eine Monomerflüssigkeit (211) als erste Ausgangskomponente lagert,
ein hohlzylinderförmiges zweites Behältnis (300) umfassend einen ersten Innenraum (320), in dem ein Knochenzementpulver (321) als zweite Ausgangskomponente lagert, und einen zweiten Innenraum (330), in den die Monomerflüssigkeit (211) förderbar ist,
ein fluidleitendes Fördermittel (340), angeordnet zwischen dem ersten Innenraum (320) und dem zweiten Innenraum (330),
wobei das erste Behältnis (200) und das zweite Behältnis (300) axial miteinander verbunden sind,
einen Austragskolben (400), angeordnet zwischen erstem Behältnis (200) und zweitem Innenraum (330)
ein im ersten Behältnis (200) angeordnetes Öffnungsmittel (220), um das Gefäß (210) bei einer ersten Bewegung des Gefäßes (210) relativ zum zweiten Behältnis (300) zu öffnen,
wobei der Austragskolben (400) für Gase und Flüssigkeiten durchlässig ausgestaltet ist, damit die Monomerflüssigkeit (211) aus dem ersten Behältnis in den zweiten Innenraum (330) fließbar ist,
wobei das erste Behältnis (200) derart in das zweite Behältnis (300) axial hineinverschiebbar ist, um
a. bei einer ersten Verschiebung (630) die Monomerflüssigkeit (211) aus dem zweiten Innenraum (330) in den ersten Innenraum (320) zu fördern, wobei der Austragskolben (400) zum Fördern der Monomerflüssigkeit eine Transformation von für Gase und Flüssigkeiten durchlässig zu für Flüssigkeiten und Feststoffe undurchlässig durchläuft,
b. bei einer zweiten Verschiebung (650) den aus der Monomerflüssigkeit (211) und dem Knochenzementpulver (321) gebildeten Knochenzement (322) aus der Vorrichtung (100) zu fördern.

8. Vorrichtung (100) nach Anspruch 7, **dadurch gekennzeichnet, dass** das erste Behältnis (200) eine Öffnungsvorrichtung (220) aufweist, um das Gefäß (210) zu öffnen und ein Ausfließen der Monomerflüssigkeit (211) zu ermöglichen.

9. Vorrichtung (100) nach Anspruch 7 oder 8, **dadurch gekennzeichnet, dass** das erste Behältnis (200) einen axial verschiebbaren Förderkolben (230) aufweist, welcher kraft- und/oder formschlüssig auf das Gefäß (210) wirkt, um das Gefäß (210) mittels einer ersten Bewegung des Gefäßes (210) auf die Öffnungsvorrichtung (220) zu pressen.

10. Vorrichtung (100) nach einem der Ansprüche 7 bis 9, **dadurch gekennzeichnet, dass** an dem ersten Behältnis (200) ein Schraubmittel (231) angeordnet ist, um den Förderkolben (230) axial zu verschieben, insbesondere, dass das Schraubmittel (231) an einem ersten Ende (202) des ersten Behältnisses (200) angeordnet ist.

11. Vorrichtung (100) nach Anspruch 10, **dadurch gekennzeichnet, dass** das Schraubmittel (231) als ein Griff ausgestaltet ist, der zumindest teilweise das erste Behältnis (200) haubenartig umfasst, insbesondere dass in der haubenartigen Umfassung ein Innengewinde (232) angeordnet ist.

12. Vorrichtung nach Anspruch 11, **dadurch gekennzeichnet, dass** zumindest Teile einer Außenfläche des ersten Behältnisses (200) ein Außengewinde (240) aufweisen, welches mit dem Innengewinde (232) der haubenartigen Umfassung kraft- und/oder formschlüssig zusammenwirkt.

13. Vorrichtung (100) nach einem der Ansprüche 7 bis 12, **dadurch gekennzeichnet, dass** ein zweites Ende (203) des ersten Behältnisses (200) kolbenartig in das zweite Behältnis (300) hineinragt.

14. Vorrichtung (100) nach Anspruch 13, **dadurch gekennzeichnet, dass** das zweite Ende (203) des ersten Behältnisses (200) kraft- und/oder form- und/oder stoffschlüssig mit einem Austragskolben (400) verbunden ist.

15. Vorrichtung (100) nach einem der Ansprüche 7 bis 14, **dadurch gekennzeichnet, dass** ein erstes Ende (301) des zweiten Behältnisses (300) ein Innengewinde (302) aufweist.

16. Vorrichtung (100) nach Anspruch 15, **dadurch gekennzeichnet, dass** zu einer zweiten Verschiebung (650) das Innengewinde (302) des zweiten Behältnisses (300) mit dem Außengewinde (240) des ersten Behältnisses (200) kraft- und/oder formschlüssig zusammenwirkt, um den Knochenzement (322) zu fördern.

17. Vorrichtung (100) nach Anspruch 16, **dadurch gekennzeichnet, dass** eine Gewindehülse (303) zwischen dem Innengewinde (302) und dem Außengewinde (240) angeordnet ist, um eine Co-axiale doppelte Gewindepaarung zu bilden.

## Claims

1. Method (600) for producing a bone cement (322) from at least two starting components in a device (100) comprising
a first hollow cylindrical container (200) and a second hollow cylindrical container (300), wherein the second container comprises a first interior (320) and a second interior (330),
a vessel (210) arranged in the first container (200), wherein the vessel (210) stores a monomer liquid (211),
a dispensing plunger (400) arranged between the first container (200) and the second interior (330),
comprising the steps of:
a. opening (610) the vessel (210) by means of a first movement of the vessel (210) relative to the second container (300),
b. the monomer liquid (211) flowing into the second interior (330), while the dispensing plunger (400) is designed to be permeable to gases and liquids,
c. conveying the monomer liquid (211) into a bone cement powder (321) stored in the first interior by means of a first axial displacement (630) of the first container (200) into the second container (300), wherein the dispensing plunger (400) for conveying the monomer liquid (211) undergoes a transformation from being permeable to gases and liquids to being impermeable to liquids and solids, and
d. squeezing the bone cement (322) formed from the monomer liquid (211) and the bone cement powder (321) out of the device (100) by means of a second axial displacement (650) of the first container (200) into the second container (300).

2. Method (600) according to claim 1, **characterized in that**, in order to open (610) according to step a), the vessel (210) is pressed onto an opening device (220) arranged in the first container (200).

3. Method (600) according to claim 2, **characterized in that**, in order to open (610) according to step a), the vessel (210) is pressed onto the opening device (220) by means of a delivery plunger (230) arranged in the first container (200).

4. Method (600) according to any one of the preceding claims, **characterized in that** a screw means (231) is arranged on the first container (200), which screw means acts in a force-fitting and/or form-fitting manner on the delivery plunger (230) to press the vessel (210) onto the opening device (220) in the first movement.

5. Method (600) according to any one of the preceding claims, **characterized in that** the first container (200) comprises a dispensing plunger (400) which is moved into the second interior (330) during the first displacement (630).

6. Method (600) according to any one of the preceding claims, **characterized in that** the dispensing plunger (400) conveys the monomer liquid (211) into the bone cement powder (321) during the first displacement (630) and squeezes the bone cement (322) out of the device during the second displacement (650).

7. Device (100) for providing a bone cement (322) made of two starting components, comprising
a hollow cylindrical first container (200) in which a vessel (210) for a monomer liquid (211) as the first starting component is stored,
a hollow cylindrical second container (300) comprising a first interior (320) in which a bone cement powder (321) is stored as the second starting component, and a second interior (330) into which the monomer liquid (211) can be conveyed,
a fluid-conducting conveying means (340) arranged between the first interior (320) and the second interior (330),
wherein the first container (200) and the second container (300) are axially connected to one another,
a dispensing plunger (400) arranged between the first container (200) and the second interior (330),
an opening means (220) arranged in the first container (200) in order to open the vessel (210) during a first movement of the vessel (210) relative to the second container (300), wherein the dispensing plunger (400) is designed to be permeable to gases and liquids so that the monomer liquid (211) can flow from the first container into the second interior (330), wherein the first container (200) can be axially displaced into the second container (300) in order
a. to convey the monomer liquid (211) from the second interior (330) into the first interior (320) during a first displacement (630), wherein the dispensing plunger (400) for conveying the monomer liquid undergoes a transformation from being permeable to gases and liquids to being impermeable to liquids and solids,
b. to convey the bone cement (322) formed from the monomer liquid (211) and the bone cement powder (321) out of the device (100) during a second displacement (650).

8. Device (100) according to claim 7, **characterized in that** the first container (200) comprises an opening device (220) in order to open the vessel (210) and to allow the monomer liquid (211) to flow out.

9. Device (100) according to claim 7 or 8, **characterized in that** the first container (200) comprises an axially displaceable delivery plunger (230), which acts on the vessel (210) in a force-fitting and/or form-fitting manner in order to press the vessel (210) onto the opening device (220) by means of a first movement of the vessel (210).

10. Device (100) according to any one of claims 7 to 9, **characterized in that** a screw means (231) is arranged on the first container (200) in order to axially displace the delivery plunger (230), in particular **in that** the screw means (231) is arranged at a first end (202) of the first container (200).

11. Device (100) according to claim 10, **characterized in that** the screw means (231) is designed as a handle which at least partially encloses the first container (200) in a hood-like manner, in particular **in that** an internal thread (232) is arranged in the hood-like enclosure.

12. Device according to claim 11, **characterized in that** at least portions of an external surface of the first container (200) comprise an external thread (240) which interacts with the internal thread (232) of the hood-like enclosure in a force-fitting and/or form-fitting manner.

13. Device (100) according to any one of claims 7 to 12, **characterized in that** a second end (203) of the first container (200) projects in a plunger-like manner into the second container (300).

14. Device (100) according to claim 13, **characterized in that** the second end (203) of the first container (200) is connected to a dispensing plunger (400) in a force-fitting and/or form-fitting and/or integrally bonded manner.

15. Device (100) according to any one of claims 7 to 14, **characterized in that** a first end (301) of the second container (300) comprises an internal thread (302).

16. Device (100) according to claim 15, **characterized in that,** for a second displacement (650), the internal thread (302) of the second container (300) interacts with the external thread (240) of the first container (200) in a force-fitting and/or form-fitting manner in order to convey the bone cement (322).

17. Device (100) according to claim 16, **characterized in that** a threaded sleeve (303) is arranged between the internal thread (302) and the external thread (240) to form a coaxial double thread pairing.

## Revendications

1. Procédé (600) de fabrication d'un ciment osseux (322) à partir d'au moins deux composants initiaux dans un dispositif (100) présentant
un premier conteneur (200) en forme de cylindre creux et un deuxième conteneur (300) en forme de cylindre creux, le deuxième conteneur présentant un premier espace intérieur (320) et un deuxième espace intérieur (330),
un récipient (210) disposé dans le premier conteneur (200), le récipient (210) stockant un liquide de monomère (211),
un piston d'extraction (400), disposé entre le premier conteneur (200) et le deuxième espace intérieur (330), comprenant les étapes :
a. d'ouverture (610) du récipient (210) au moyen d'un premier mouvement du récipient (210) par rapport au deuxième conteneur (300),
b. d'écoulement du liquide de monomère (211) dans le deuxième espace intérieur (330), pendant que le piston d'extraction (400) est conçu perméable aux gaz et aux liquides,
c. de transport du liquide de monomère (211) dans une poudre de ciment osseux (321) stockée dans le premier espace intérieur au moyen d'un premier déplacement axial (630) du premier conteneur (200) dans le deuxième conteneur (300), le piston d'extraction (400) passant, pour le transport du liquide de monomère (211), par une transformation de perméable aux gaz et aux liquides à imperméable aux liquides et aux solides, et
d. de pressage du ciment osseux (322) formé à partir du liquide de monomère (211) et de la poudre de ciment osseux (321) hors du dispositif (100) au moyen d'un deuxième déplacement axial (650) du premier conteneur (200) dans le deuxième conteneur (300).

2. Procédé (600) selon la revendication 1, **caractérisé en ce que**, pour l'ouverture (610) selon l'étape a), le récipient (210) est pressé sur un dispositif d'ouverture (220) disposé dans le premier conteneur (200).

3. Procédé (600) selon la revendication 2, **caractérisé en ce que**, pour l'ouverture (610) selon l'étape a), le récipient (210) est pressé sur le dispositif d'ouverture (220) au moyen d'un piston de transport (230) disposé dans le premier conteneur (200).

4. Procédé (600) selon l'une quelconque des revendications précédentes, **caractérisé en ce qu'**un moyen de vissage (231) est disposé sur le premier conteneur (200), lequel agit par engagement de force et/ou de forme sur le piston de transport (230), pour presser le récipient (210) lors du premier mouvement sur le dispositif d'ouverture (220).

5. Procédé (600) selon l'une quelconque des revendications précédentes, **caractérisé en ce que** le premier conteneur (200) présente un piston d'extraction (400), lequel est déplacé dans le deuxième espace intérieur (330) lors du premier déplacement (630).

6. Procédé (600) selon l'une quelconque des revendications précédentes, **caractérisé en ce que** le piston d'extraction (400) transporte, lors du premier déplacement (630), le liquide de monomère (211) dans la poudre de ciment osseux (321) et presse, lors du deuxième déplacement (650), le ciment osseux (322) hors du dispositif.

7. Dispositif (100) de fourniture d'un ciment osseux (322) à partir de deux composants initiaux, présentant
un premier conteneur (200) en forme de cylindre creux, dans lequel est stocké un récipient (210) pour un liquide de monomère (211) comme premier composant initial,
un deuxième conteneur (300) en forme de cylindre creux comprenant un premier espace intérieur (320), dans lequel est stockée la poudre de ciment osseux (321) comme deuxième composant initial, et un deuxième espace intérieur (330), dans lequel le liquide de monomère (211) peut être déplacé,
un moyen de transport guidant les fluides (340), disposé entre le premier espace intérieur (320) et le deuxième espace intérieur (330),
le premier conteneur (200) et le deuxième conteneur (300) étant liés axialement l'un à l'autre, un piston d'extraction (400), disposé entre le premier conteneur (200) et le deuxième espace intérieur (330)
un moyen d'ouverture (220) disposé dans le premier conteneur (200), pour ouvrir le récipient (210) lors d'un premier mouvement du récipient (210) par rapport au deuxième conteneur (300),
le piston d'extraction (400) étant conçu perméable aux gaz et aux liquides, pour que le liquide de monomère (211) puisse s'écouler du premier conteneur dans le deuxième espace intérieur (330),
le premier conteneur (200) pouvant être poussé axialement dans le deuxième conteneur (300) de façon à
a. lors d'un premier déplacement (630), transporter le liquide de monomère (211) du deuxième espace intérieur (330) dans le premier espace intérieur (320), le piston d'extraction (400) passant, pour le transport du liquide de monomère, par une transformation de perméable aux gaz et aux liquides à imperméable aux liquides et aux solides,
b. lors d'un deuxième déplacement (650), transporter le ciment osseux (322) formé à partir du liquide de monomère (211) et de la poudre de ciment osseux (321) hors du dispositif (100).

8. Dispositif (100) selon la revendication 7, **caractérisé en ce que** le premier conteneur (200) présente un dispositif d'ouverture (220) pour ouvrir le récipient (210) et permettre l'écoulement du liquide de monomère (211).

9. Dispositif (100) selon la revendication 7 ou 8, **caractérisé en ce que** le premier conteneur (200) présente un piston de transport (230) déplaçable axialement, lequel agit par engagement de force et/ou de forme sur le récipient (210), pour presser le récipient (210) au moyen d'un premier mouvement du récipient (210) sur le dispositif d'ouverture (220).

10. Dispositif (100) selon l'une quelconque des revendications 7 à 9, **caractérisé en ce qu'**un moyen de vissage (231) est disposé sur le premier conteneur (200), pour déplacer le piston de transport (230) axialement, en particulier **en ce que** le moyen de vissage (231) est disposé à une première extrémité (202) du premier conteneur (200).

11. Dispositif (100) selon la revendication 10, **caractérisé en ce que** le moyen de vissage (231) est conçu comme une poignée, qui entoure au moins partiellement sous forme de couvercle le premier conteneur (200), en particulier **en ce qu'**un filetage intérieur (232) est disposé dans l'enceinte en forme de couvercle.

12. Dispositif selon la revendication 11, **caractérisé en ce qu'**au moins des parties d'une surface extérieure du premier conteneur (200) présente un filetage extérieur (240), lequel interagit avec le filetage intérieur (232) de l'enceinte en forme de couvercle.

13. Dispositif (100) selon l'une quelconque des revendications 7 à 12, **caractérisé en ce qu'**une deuxième extrémité (203) du premier conteneur (200) fait saillie à la façon d'un piston dans le deuxième conteneur (300).

14. Dispositif (100) selon la revendication 13, **caractérisé en ce que** la deuxième extrémité (203) du premier conteneur (200) est liée par engagement de force et/ou de forme et/ou de matière à un piston d'extraction (400).

15. Dispositif (100) selon l'une quelconque des revendications 7 à 14, **caractérisé en ce qu'**une première extrémité (301) du deuxième conteneur (300) présente un filetage intérieur (302).

16. Dispositif (100) selon la revendication 15, **caractérisé en ce que**, pour un deuxième déplacement (650), le filetage intérieur (302) du deuxième conteneur (200) interagit par engagement de force et/ou de forme avec le filetage extérieur (240) du premier conteneur (200) pour transporter le ciment osseux (322).

17. Dispositif (100) selon la revendication 16, **caractérisé en ce qu'**une douille filetée (303) est disposée entre le filetage intérieur (302) et le filetage extérieur (240), pour former une double paire de filetages co-axiale.
